Europäisches Patentamt

European Patent Office

Office européen des brevets

(11) Publication number: **0 242 956**
A1

## EUROPEAN PATENT APPLICATION

(21) Application number: 87301671.1

(22) Date of filing: 25.02.87

(51) Int. Cl.³: **A 61 M 5/28**
A 61 M 5/315, A 61 M 5/00

(30) Priority: 26.02.86 US 833049

(43) Date of publication of application:
28.10.87 Bulletin 87/44

(84) Designated Contracting States:
AT BE CH DE ES FR GB GR IT LI LU NL SE

(71) Applicant: INTELLIGENT MEDICINE, INC.
6665 South Kenton Street
Englewood Colorado 80111(US)

(72) Inventor: Howson, David Crampton
253 Adams Street
Denver Colorado 80206(US)

(74) Representative: Bizley, Richard Edward et al,
BOULT, WADE & TENNANT 27 Furnival Street
London EC4A 1PQ(GB)

(54) Self-contained material mixing apparatus.

(57) A self-contained material mixing apparatus (12) is disclosed that is particularly useful for mixing two substances separately contained in the apparatus (12) until an actuator is moved within the apparatus (12) to cause one of the substances to be brought into contact with the other substance. The apparatus (12) may be embodied as a syringe having a piston (16) therein separating two compartments (18, 19) within the syringe body (14). The piston (16) causes an incompressible substance in the rearward compartment (19) to pass to the forward compartment (18) when the volume of the rearward compartment (19) is reduced, but precludes passage of substances in the forward compartment (18) to the rearward compartment (19). The substances to be ultimately mixed together are separately placed in the compartments (18, 19), which substances can be, for example, a drug and a diluent (one of which is preferably in liquid form). For mixing of the substances, the piston (16) is moved rearwardly in the syringe (12) and this results in incompressible material in the rearward compartment (19) being forced to the fron compartment (18) through the one-way valving provided by the piston (16). After mixing of the substances in the forward compartment (18), the material is discharged from the syringe (12) to a patient by moving the piston (16) forwardly in the syringe (12) to expel the mixture from the syringe (12) through an outlet nozzle located at the front of the syringe (12).

Fig_1

1

## SELF-CONTAINED MATERIAL MIXING APPARATUS

This invention relates to a material mixing apparatus and, more particularly, relates to a self-contained drug mixing apparatus.

It is well known that various substances can, or must, be maintained separated until the time of use, and various devices have been heretofore suggested and/or utilized to accomplish this end. With respect to drugs, it is well known that at least some drugs, when in the delivery stage, have a short shelf-life and/or cannot be mixed with other substances until quite near the time of intended use.

Various devices have been heretofore suggested and/or utilized for mixing drugs stored in separate receptacles prior to use (see, for example, U.S. Patent Nos. 3,938,520 and 3,125,092), and some such devices have utilized a closed system wherein the drug was introduced into a diluent within a bag in the device (see, for example, U.S. Patent No. 4,410,321).

It has also been heretofore suggested that a syringe could have a substance stored therein, with the syringe, when inserted into a container, withdrawing a liquid substance to be mixed with the substance stored in that container (see, for example, U.S. Patent No. 3,337,041). In addition, a double compartment ampule has also been suggested for separately storing two substances with the substances being separated by a divider that when displaced allowed mixing of the substances (see, for example, U.S. Patent No. 1,929,616).

Thus, while devices have been heretofore suggested and/or utilized for mixing substances, such devices have not proven to be completely successful in providing a mixing device wherein the substances can be separately stored until mixing within the device, with the device then being utilized to deliver the mixture to a patient, which delivery can be through a controlled drive so that the device can deliver individual doses.

This invention provides a device for mixing substances, particularly drugs and associated materials, wherein the substances are combined within the mixing and delivery unit until when an actuator within the unit is moved, after which a mixture of the substances is discharged from the device by again moving the actuator. The actuator of the device is provided with means which permit liquid to flow from a compartment on one side of the actuator to a compartment on the other side but not vice-versa, so that in use liquid from one compartment can be forced into the other compartment, in which there is another liquid or a solid, and the two substances can then be discharged as a mixture. (The term "mixture" includes solutions).

This invention is further defined by the appended claims. Changes in the precise embodiment of the herein disclosed invention are meant to be included within the scope of the invention.

The accompanying drawings illustrate complete embodiments of the invention according to the best mode so far devised for the practical application of the principles thereof, and in which:

FIGURE 1 is a perspective of the material mixing apparatus of this invention shown embodied as a syringe having separate compartments therein separated by the syringe piston;

FIGURE 2 is a sectional view taken through lines 2-2 of FIGURE 1;

FIGURE 3 is an exploded view of the device shown in FIGURES 1 and 2;

FIGURE 4 is a sectional view taken through lines 4-4 of FIGURE 2;

FIGURE 5 is a sectional view similar to that of FIGURE 4 and illustrating movement of incompressible material from the rearward compartment to the forward compartment upon movement of the piston rearwardly in the syringe;

FIGURE 6 is a sectional view taken through lines 6-6 of FIGURE 2;

FIGURE 7 is a sectional view taken through lines 7-7 of FIGURE 2;

FIGURE 8 is a exploded view of an alternate embodiment of a syringe piston assembly that may be utilized in this invention;

FIGURE 9 is an exploded view similar to that of FIGURE 8 and illustrating another alternate embodiment of a syringe piston assembly that may be utilized in this invention; and

FIGURE 10 is a sectional view similar to that of FIGURES 4 and 5 and illustrating yet another alternate embodiment of the syringe piston that may be utilized in this invention.

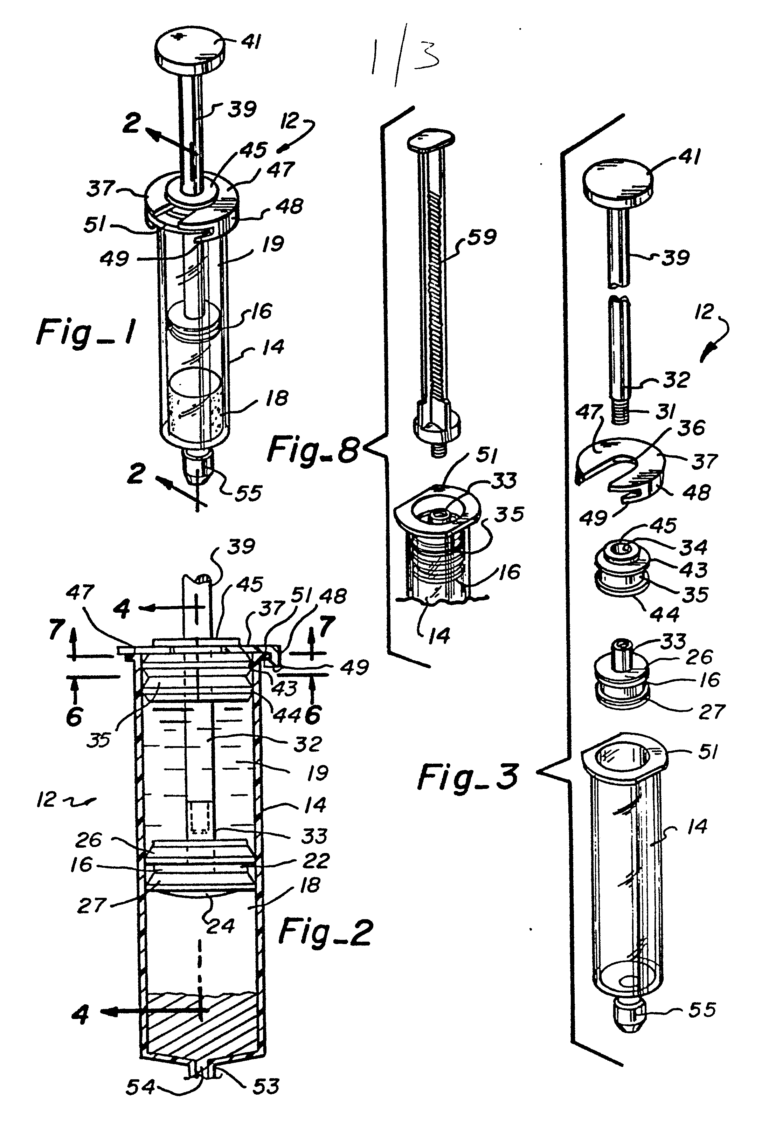

Referring to the drawings, the material mixing apparatus 12 of this invention is best shown overall, embodied as a syringe, by the perspective view of FIGURE 1, along with the sectional view of FIGURE 2 and the exploded view of FIGURE 3. As shown, syringe 12 has a cylindrical syringe body 14, and a piston 16 positioned within syringe body 14 so that separate forward and rearward compartments 18 and 19, respectively, are formed at opposite sides of the piston.

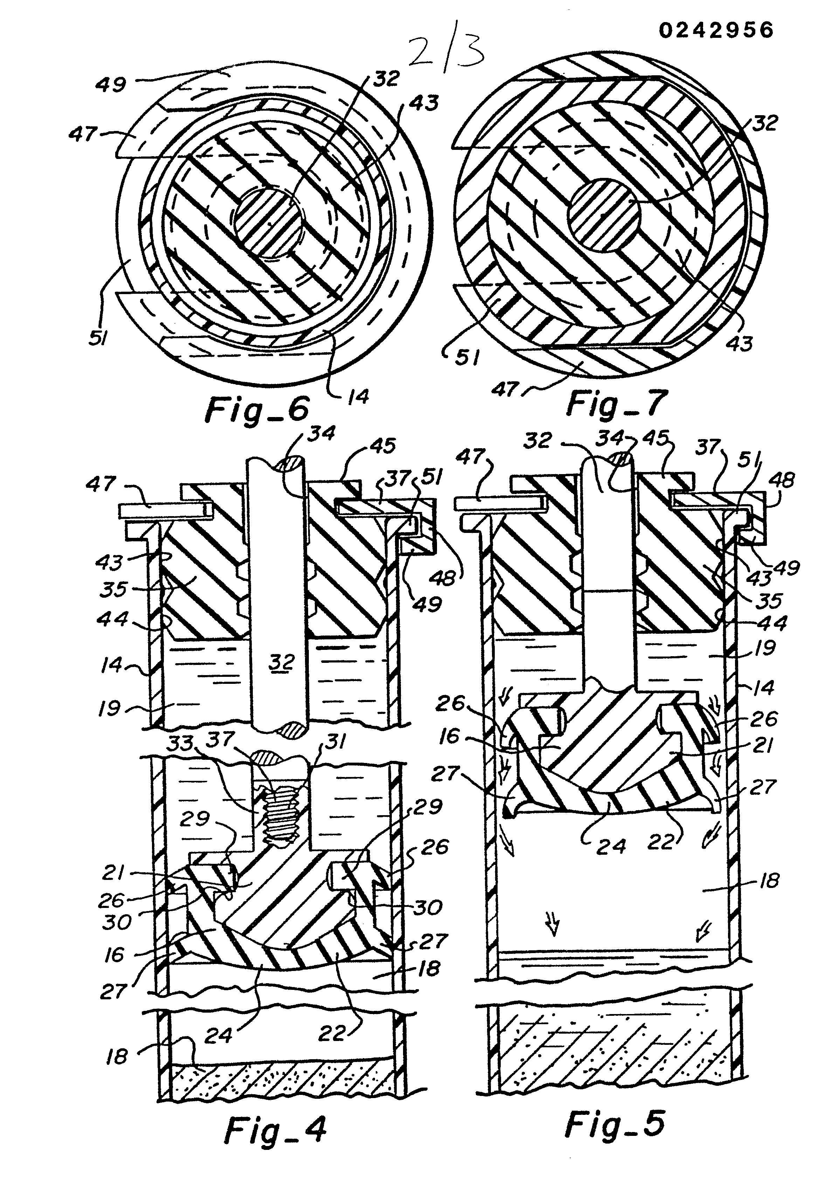

Piston 16, as best shown in FIGURES 4 and 5, includes a cylindrical base portion 21 having one-way valve 22 mounted thereon. Valve 22 includes a bottom portion 24 bridging the bottom of base portion 21, a pair of spaced annular shoulders 26 and 27 formed of resilient material, such as rubber, which engage the inner walls of syringe body 14, and annular mounting shoulder 29 which is received in notch 30 of piston base portion 21.

The free ends of shoulders 26 and 27 engage the inner wall of syringe body 14 at an angle that forms one-way valving for allowing passage of materials from rearward compartment 19 to forward compartment 18 by wedging the shoulders inwardly (away from the inner wall of syringe body 14) under pressure to allow materials in the rearward compartment to pass between the inner wall of the syringe body and the shoulders and thus move into the forward compartment.

Movement of materials from the rear compartment to the front compartment is precluded, however, since pressure on materials in the forward compartment, tending to move such materials toward the rear compartment, causes the shoulders to be more tightly wedged against the inner wall of the syringe body thus precluding movement of material in the rearward direction from the forward compartment to the rearward compartment.

Piston 16 is connected to one end 31 (having screw threads thereon) of piston rod 32 by means of annular fastening stud 33 (centrally mounted to the rear face of base portion 21 of piston 16 and having internal screw threads therein) so that piston rod 32 controls actuation of piston 16 in opposite axial directions within syringe body 14 to vary the volume of compartments 18 and 19 formed at the opposite sides of piston 16.

Piston rod 32 extends rearwardly from piston 16 and passes from syringe body 14 through aperture 34 in a second piston, or plug, 35 and notch 36 of safety clip 37 so that the rearmost portion 39 of rod 32 is external of syringe body 14. Disk 41 may be mounted at the rear end of rod 32.

Plug 35 is similar to piston 16 and includes a pair of shoulders 43 and 44 that extend to the inner wall of syringe body 14 to form a seal thereat. Unlike shoulders 26 and 27, however, shoulders 43 and 44 do not form a one-way valve, but instead, the shoulders are sufficiently rigid to engage the inner wall of the syringe body to thereby preclude passage of materials, in either direction, past plug 35 when positioned within syringe body 14. In addition, an annular resilient washer 45 extends upwardly from the rear side of plug 35 to receive a portion of safety clip 37 partially therearound.

Piston rod 32 is axially movable within aperture 34 of plug 35 to allow rearward movement of the rod, and is also preferably movable through aperture 34 to allow forward movement of the rod (although alternately, plug 35 can be constrained to movement with rod 32 after rod 32 has been moved to the rearmost position).

Safety clip 37 is a spring type clip that is mounted at the rear end of body portion 14. As shown in FIGURES 1, 2 and 4 through 7, clip 37 includes annular washer 47 the inner wall of which engages and is received in a notch in the outer wall of resilient washer 45 (the inner wall of which washer engages piston 32). Washer 47 has a depending flange 48 around a major portion thereof, and flange 48 has an inwardly directed lip 49 thereon so that the outwardly directed lip 51 of syringe body 14 is received between disk 47 and lip 49. Safety clip 37 may be formed of plastic material so that the clip can be positioned by being snapped into place, as is well known.

An outlet, or nozzle, 53 is provided at the forward end of syringe body 14, and outlet 53 has an aperture 54 therein that is in communication with forward compartment 18. In order to seal the forward compartment, a cap 55 is provided, as indicated in FIGURES 1 and 3.

Prior to mixing, piston 16 provides a seal between compartments 18 and 19 so that the compartments separately store substances to be ultimately mixed, which substances can be, for example, a solid drug material in forward compartment 18 and a diluent for reconstituting the drug in rearward compartment 19.

In operation to mix the stored substances, piston 16 is first moved rearwardly (i.e., in an axial direction away from the outlet 53 at the front of the syringe). This decreases the volume of rearward compartment 19 and enlarges the volume of forward compartment 18, which increases the pressure in the rearward compartment. By providing an incompressible material in the rearward compartment (such as a diluent, which can be water, for example), the incompressible material is forced, under pressure, against shoulder 26 (and then against shoulder 27). This wedges the edges of the shoulders away from the inner wall of syringe body 14 so that the material passes from the rearward compartment to the forward compartment to mix with the substances stored thereat. Thus, if a drug material in solid form is stored at the forward compartment and a liquid diluent is forced from the rearward compartment to the forward compartment, the diluent mixes with the drug to reconstitute the drug at the forward compartment.

When the piston has been moved rearwardly to its fullest extent, the volume of the rearward compartment is at a minimum (near zero), and the volume at the forward compartment is at a maximum. The piston is precluded, or stopped, from moving out of syringe body 14 by safety clip 37 and plug 35. After mixing is completed (as by shaking the syringe, for example), the syringe is ready for delivery of the mixture to a patient. This is affected by moving the piston forwardly within the syringe body (the one-way valving precludes movement of the mixture to the rearward compartment) to cause the mixture to be discharged through aperture 54 of outlet 53. While not specifically shown, it is to be realized that outlet 53 can be connected with a patient through a suitable conduit and needle arrangement, as is well known.

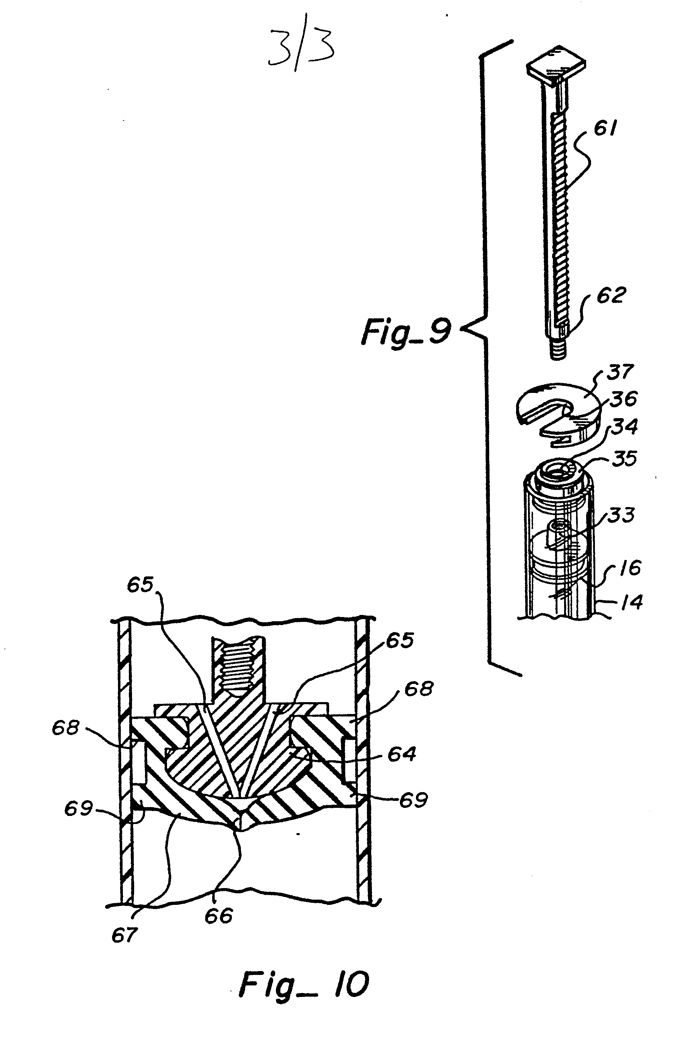

An alternate embodiment of the piston actuating apparatus is shown in FIGURE 8. As shown, piston rod 32 has been replaced by drive rod 59, which rod connects with plug 35 which is connected to piston 16 so that the plug and piston are moved together forwardly in the syringe to effect delivery of drug (rod 59 replaces rod 32 only after mixing has occurred by moving piston 16 rearwardly with the syringe body and clip 37 has been removed). Drive rod 59 is adapted for use with a control mechanism so that the rod can be driven as needed to affect drug delivery. A control mechanism and associated drive rod for driving the rod in a programmed manner is described in European Patent Application No. 86106427.7 published December 17, 1986.

FIGURE 9 illustrates another embodiment of the piston assembly similar to that shown in FIGURE 8. As shown in this embodiment, however, drive rod 61 is utilized with rod 61 being configured at inner end 62 to pass through aperture 34 in plug 35 and notch 36 in clip 37, and then be directly connected to piston 16 to move only the piston forwardly to effect drug delivery after mixing has occurred.

FIGURE 10 illustrates an alternate embodiment of the piston assembly which provide an alternate manner of providing the needed one-way valve. As shown, base 64 of the piston has apertures 65 therein leading to one-way valve 66 formed in the central portion of bridging section 67. As shown, materials can be forced, under pressure, from the rearward compartment to the forward compartment, but the valve structure precludes movement of materials rearwardly from the forward compartment to the rearward compartment. When embodied in this manner, shoulders 68 and 69 extend to the inner wall of the syringe body at a right angle to form a seal thereat and do not allow transfer of materials in either direction between the inner wall of the syringe body and the piston.

As can be appreciated from the foregoing, this invention provides an improved apparatus for mixing substances separately stored in the apparatus with mixing and delivery being effected by an actuator within the apparatus.

CLAIMS:

1. A mixing apparatus, comprising:

a storage receptacle defining a chamber that includes first and second compartments, in use said first compartment having a first substance therein and said second compartment having a second substance therein, said storage receptable having an opening at one portion communicating with said second compartment;

movable means within said chamber between said first and second compartments, said movable means including valving means having a substance passing portion angularly oriented with respect to the direction of movement of said movable means within said chamber with said substance passing portion being deflectable due to pressure exerted thereon by said first substance to thereby permit passage of said first substance from said first compartment to said second compartment upon application of pressure to said first substance but precluding passage of said first and second substances from said second compartment to said firest compartment; and

actuating means having an elongated portion attached to said movable means and restricted to common movement therewith so that movement of said elongated portion in one longitudinal direction causes movement of said movable means in a first direction to cause pressure to be applied to said first substance so that said first substance is transferred from said first compartment to said second compartment for mixing thereat, and movement of said elongated portion in the other opposite longitudinal direction causes movement of said movable means in a second direction to cause a mixture of said first and second substances to be discharged from said apparatus through said opening.

2. The apparatus of claim 1 wherein said storage receptacle is a syringe having a body portion forming said chambers, and wherein said movable means is a piston within said body portion.

3. The apparatus of either claim 1 or 2 wherein said apparatus includes stop means for determining the limit of movement of said movable means for determining the limit of movement of said movable means in said first direction.

4. The apparatus of claim 3 wherein said stop means precludes movement of said movable means outside said chamber.

5. The device of either of claims 3 or 4 wherein said stop means includes retaining means having aperture means for receiving said actuating means therethrough whereby said movable means may be actuated externally of said syringe.

6. The apparatus of any of claims 1 through 5 wherein said mixing apparatus is for mixing a drug, and wherein at least one of said first and second compartments has said drug therein, whereby said drug is mixed with a substance in the other of said first and second compartments upon movement of said movable means in said one direction, and whereby said drug mixture is expelled from said compartment upon movement of said movable means in said second direction.

7. The apparatus of claim 6 wherein said drug is in one of a solid state and a liquid state, and wherein said diluent is in the other of said solid state and liquid state.

8. The apparatus of claim 7 wherein said substance in the other of said first and second compartments is a liquid diluent, and wherein said drug is reconstituted by said mixing prior to delivery from said apparatus.

9. The apparatus of any of claims 2 thorugh 8 wherein said substance passing portion of said movable means of said piston includes annular shoulder means extending from said piston and engaging the wall of said syringe body portion, said shoulder means being formed so that upon movement of the piston in the first direction said first substance in said first compartment can terminate engagement of said shoulder means with said wall means to allow passage of said first substance therebetween.

10. The apparatus of claim 9 wherein said shoulder means is formed so that upon movement of the piston in the second direction substances in said second compartment cause engagement of said shoulder means with the wall of said syringe body portion to thereby preclude passage of said substances from said second compartment to said first compartment.

11. The apparatus of any of claims 2 through 8 wherein said substance passing portion of said valving means of said piston includes relatively movable elements positioned at one side of said piston, with said piston having apertures communicating therethrough to said relatively movable elements of said valving means whereby said relatively movable elements are separated upon application of pressure to said first substance in said first compartment to allow passage of said first substance through said valving means, and whereby said relatively movable elements are precluded from separating upon application of pressure to substances in said second compartment to preclude passage of substances in said second compartment to said first compartment.

12. The apparatus of any of claims 1 through 11 wherein said elongated portion of said actuating means includes first and second elongated sections and cooperating attachment means mounted at one end of each said section whereby said section not connected to said piston can be replaced with a drive rod adapted to be driven in a predetermined manner.

13. A syringe comprising a plug or other sealing means sealing the end of the syringe through which the piston rod or drive rod passes to prevent escape of liquid through said end, the piston having one-way valve means to permit liquid flow from an upper chamber between the piston and the sealing means to a lower chamber between the piston and the outlet end of the syringe when the piston is moved upwards but not to permit liquid flow in the opposite direction.

0242956

1/3

Fig_1

Fig_8

Fig_3

Fig_2

Fig_6

Fig_7

Fig_4

Fig_5

0242956

3/3

*Fig_9*

61

62

37
36
34
35
33
16
14

65

65

68

68

64

69

69

67

66

*Fig_ 10*

# EUROPEAN SEARCH REPORT

## DOCUMENTS CONSIDERED TO BE RELEVANT

EP 87301671.1

| Category | Citation of document with indication, where appropriate, of relevant passages | Relevant to claim | CLASSIFICATION OF THE APPLICATION (Int Cl 4) |
|---|---|---|---|
| X | US - A - 3 511 239 (L.TUSCHHOFF) | 1-10, 13 | A 61 M 5/28 |
| Y | * Totality; especially column 3, lines 8-16; column 3, line 48 - column 4, line 9; claim 1 * | 12 | A 61 M 5/315 <br> A 61 M 5/00 |
| | -- | | |
| X | US - A - 3 699 961 (R.SZPUR) | 1-10, 13 | |
| Y | * Totality; especially fig. 5-17; abstract; column 2, lines 10-47 * | 12 | |
| | -- | | |
| Y | US - A - 4 371 094 (CH.G.HUTTER III) | 12 | |
| | * Fig. 3,4,9,13; abstract * | | TECHNICAL FIELDS SEARCHED (Int Cl 4) |
| | -- | | |
| X | US - A - 3 662 753 (K.B.TASSELL) | 1-10, 13 | A 61 J 1/00 |
| | * Totality * | | A 61 M 5/00 |
| | -- | | B 65 D 81/00 |
| Y | CH - A - 445 721 (N.BAY-SCHMITH) | 1-8,11 | B 65 D 83/00 |
| X | * Totality * | 13 | |
| | -- | | |
| Y | DE - A1 - 3 208 472 (F.WILLICH GMBH) | 1-8, 11 | |
| | * Fig. 1; page 10, paragraph 2 - page 11, paragraph 1; abstract * | | |
| | -- | | |

The present search report has been drawn up for all claims

| Place of search | Date of completion of the search | Examiner |
|---|---|---|
| VIENNA | 22-07-1987 | LUDWIG |

# EUROPEAN SEARCH REPORT

## DOCUMENTS CONSIDERED TO BE RELEVANT

EP 87301671.1

| Category | Citation of document with indication, where appropriate, of relevant passages | Relevant to claim | CLASSIFICATION OF THE APPLICATION (Int. Cl 4) |
|---|---|---|---|
| A | US - A - 4 479 578 (D.J.BRIGNOLA et al.) | 1 | |
| X | * Totality; especially fig. 13-17; column 7, lines 8-50 * | 13 | |
| | -- | | |
| A | FR - A - 2 082 128 (H.R.ROBERT) | 1 | |
| X | * Fig. 1; page 2, line 31 - page 5, line 21 * | 13 | |
| | ---- | | |

TECHNICAL FIELDS SEARCHED (Int. Cl.4)

The present search report has been drawn up for all claims

| Place of search | Date of completion of the search | Examiner |
|---|---|---|
| VIENNA | 22-07-1987 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82